# FASCICULE DE BREVET EUROPEEN

(11) **EP 1 233 750 B1**
(45) Date de publication et mention de la délivrance du brevet: **11.08.2004**
(21) Numéro de dépôt: 99956094.9
(22) Date de dépôt: 19.11.1999
(51) Int. Cl.: A61K 7/50, A61K 7/48, A61K 7/02, A61K 7/40

(54) **LATEX INVERSE ET UTILISATION EN COSMETIQUE**
INVERSER LATEX UND VERWENDUNG IN DER KOSMETIK
NOVEL POSITIVE LATEX AND USE IN COSMETICS

(43) Date de publication de la demande: 28.08.2002
(73) Titulaire: SOCIETE D'EXPLOITATION DE PRODUITS POUR LES INDUSTRIES CHIMIQUES, S.E.P.P.I.C., 75321 Paris Cédex 07 (FR)
(72) Inventeur: MALLO, Paul, F-78400 Chatou (FR); MICHEL, Nelly, F-94700 Maisons Alfort (FR)
(74) Mandataire: Conan, Philippe Claude
(86) Numéro de dépôt international: PCT/FR1999/002850
(87) Numéro de publication internationale: WO 2001/035922

(56) Documents cités:
- EP-A- 0 503 853
- EP-A- 0 796 614
- DE-A- 19 523 596
- FR-A- 2 681 245
- FR-A- 2 698 004
- FR-A- 2 701 844
- US-A- 4 873 078
- US-A- 5 879 718
- US-A- 5 885 563

## Description

La présente demande concerne des latex eau dans huile épaississants, leur procédé

de préparation et leur application en tant qu'épaississant et/ou émulsionnant pour des produits de soins de la peau et des cheveux ou pour la fabrication de préparations cosmétiques, dermopharmaceutiques ou pharmaceutiques.

Différents épaississants existent et sont déjà utilisés pour ces usages. On connaît en particulier les produits naturels tels que les gommes de guar ou l'amidon mais dont les inconvénients sont ceux inhérents aux produits naturels, tels que la fluctuation des cours, les difficultés d'approvisionnement et une qualité aléatoire.

Les polymères synthétiques sous forme de poudre, principalement les polyacides acryliques sont également largement utilisés mais présentent l'inconvénient de nécessiter une neutralisation lors de l'utilisation, car ils ne développent leur viscosité qu'à partir d'un pH supérieur à 6.5 et leur mise en solution est souvent fastidieuse.

Il existe aussi des polymères épaississants synthétiques, se présentant sous forme de latex inverses, c'est-à-dire dont la phase continue est une huile. La mise en solution de ces latex est extrêmement rapide ; les polymères contenus dans ces latex inverses sont le plus souvent des copolymères acrylamide / acrylate de métal alcalin ou acrylamide/acrylamido 2-méthyl 2-propanesulfonate de sodium ; ils sont déjà neutralisés et, lorsqu'ils sont mis en solution dans l'eau, par exemple à une concentration de 1%, on observe que le pH est généralement supérieur à 6.

Les copolymères acrylamide/acrylate de sodium ne développent cependant pas de propriétés épaississantes importantes lorsqu'on abaisse le pH en dessous de 6 ; par contre les copolymères acrylamide/acrylamido 2-méthyl 2-propanesulfonate de sodium décrits dans EP 0 503 853, gardent une capacité épaississante importante même à pH égal à 4. Cependant, les latex inverses engendrent parfois des réactions d'intolérance de certaines peaux sensibles ; la demanderesse s'est donc intéressée à la recherche de nouvelles émulsions inverses fluides comprenant les polymères cités précédemment, qui soient mieux tolérées par la peau que ceux de l'état de la technique et à leur utilisation dans la préparation de compositions cosmétiques, dermopharmaceutiques ou pharmaceutiques.

Les compositions cosmétiques détergentes décrites dans EP-A-0 796 614 contiennent un polyacrylamide épaississant, des tensioactifs, et sous forme d'émulsions eau-dans-huile, un mélange d'hydrocarbones isoparaffiniques en C₁₂-C₁₃.

C'est ainsi qu'elle a préparé une nouvelle composition comprenant une phase huile, une phase aqueuse, au moins un agent émulsifiant de type eau dans huile (E/H), au moins un agent émulsifiant de type huile dans eau (H/E), sous forme d'un latex inverse comprenant de 20% à 60% en poids, et de préférence de 30% à 50% en poids, d'un polyélectrolyte anionique, branché ou réticulé, à base d'acide 2-méthyl 2-[(1-oxo 2-propènyl) amino] 1-propanesulfonique partiellement ou totalement salifié, copolymérisé avec l'acrylamide, le solvant organique constitutif de la phase huile étant l'isohexadécane

L'isohexadécane, qui est identifié dans Chemical Abstracts par le numéro RN = 93685-80-4, est un mélange d'isoparaffines en C₁₂, C₁₆ et C₂₀ contenant au moins 97% d'isoparaffines en C₁₆, parmi lesquelles le constituant principal est le 2,2,4,4,6,8,8-heptaméthyl nonane (RN = 4390-04-9). Il est commercialisé en France par la société Bayer.

Le polyélectrolyte anionique compris dans le latex inverse tel que défini précédemment, comporte en proportions molaires de 30% à 50% de sel de sodium ou de sel d'ammonium de l'acide 2-méthyl 2-[(1-oxo 2-propènyl) amino] 1-propanesulfonique et de 50% à 70% d'acrylamide, et est notamment réticulé et/ou branché avec un composé diéthylénique ou polyéthylénique dans la proportion molaire exprimée par rapport aux monomères mis en oeuvre, de 0,005% à 1 %, et de préférence de 0,01% à 0,2%. L'agent de réticulation et/ou l'agent de ramification est plus particulièrement choisi parmi le méthacrylate d'éthylène glycol, le diallyloxyacétate de sodium, le diacrylate d'éthylène glycol, le diallyl urée, le triméthylol propanetriacrylate ou le méthylène bis(acrylamide).

Le latex inverse tel que défini ci-dessus contient généralement de 4% à 10% en poids, d'agents émulsifiants. Sa phase huile représente de 15% à 40%, et de préférence de 20% à 25%, de son poids total. Ce latex peut en outre contenir un ou plusieurs additifs choisis notamment parmi les agents complexant, les agents de transfert ou les agents limiteurs de chaînes.

L'invention a donc pour objet, une composition cosmétique, dermopharmaceutique ou pharmaceutique, caractérisée en ce qu'elle comprend au moins un composé épaississant, au moins un latex inverse tel que défini précédemment.

La composition cosmétique, dermopharmaceutique ou pharmaceutique définie ci-desssus comprend généralement de 0,1 % à 10 % et plus particulièrement entre 0,5% et 5% en poids dudit latex inverse. Elle se présente notamment, sous la forme d'un lait, d'une lotion, d'un gel, d'une crème, d'un savon, d'un bain moussant, d'un baume, d'un shampooing ou d'un après shampooing.

De façon générale, ledit latex inverse, peut remplacer avantageusement les produits vendus sous le nom SEPIGEL™ 305 ou SEPIGEL™ 501 par la demanderesse, dans les compositions cosmétiques, dermopharmaceutiques ou pharmaceutiques, car il présente aussi une bonne compatibilité avec les autres excipients utilisés pour la préparation de formulations telles que les laits, les lotions, les crèmes, les savons, les bains, les baumes, les shampooings ou les après shampooings. Il peut aussi être utilisé en combinaison lesdits SEPIGEL
Il est notamment compatible avec les concentrés décrits et revendiqués dans les publications internationales WO 92/06778, WO 95/04592, W095/13863 ou FR 2734 496, ou avec les agents tensioactifs décrits dans WO 93/08204.

Il est particulièrement compatible avec le MONTANOV™ 68, le MONTANOV™ 82, le MONTANOV™ 202 ou le SEPIPERL™ N. Il peut également être utilisé dans des émulsions du type de celles décrites et revendiquées dans EP 0 629 396 et dans les dispersions aqueuses cosmétiquement ou physiologiquement acceptables avec un composé organo-polysiloxane choisi, par exemple parmi ceux décrits dans WO 93/05762 ou dans WO 93/21316. Il peut également être utilisé pour former des gels aqueux à pH acide cosmétiquement ou physiologiquement acceptables, tels que ceux décrit dans WO 93/07856 ; il peut encore être utilisé en association avec des celluloses non-ioniques, pour former par exemple des gels de coiffage, tels que ceux décrits dans EP 0 684 024, ou encore en association avec des esters d'acides gras et de sucre, pour former des compositions pour le traitement du cheveux ou de la peau telles que celles décrites dans EP 0 603 019. ou encore dans les shampooings ou après shampooings tels que décrits et revendiqués dans WO 92/21316 ou enfin en association avec un homo polymère anionique tels que le CARBOPOL™ pour former des produits de traitement des cheveux comme ceux décrits dans DE 195 23596. Il est également compatible avec de nombreux principes actifs, tels que par exemple, les agents autobronzants comme le dihydroxyacétone (DHA) ou les agents anti-acné ; il peut donc être introduit dans des compositions auto-bronzantes comme celles revendiquées dans EP 0 715 845, EP 0604249, EP 0576188 ou dans WO 93/07902. Il est également compatible avec les dérivés N-acylés d'aminoacides, ce qui permet son utilisation dans des compositions apaisantes notamment pour peaux sensibles, telles que celles décrites ou revendiquées dans WO 92/21318, WO 94/27561 ou WO 98/09611. Il est aussi compatible avec les acides glycoliques, avec l'acide lactique, avec l'acide salicylique les rétinoïdes, le phénoxy éthanol, les sucres, le glycéraldéhyde, les xanthanes, les acides de fruit, et les divers polyols utilisés dans la fabrication de formulations cosmétiques.

L'invention a donc aussi pour objet, l'utilisation d'un latex inverse tel que défini précédemment, pour préparer une composition cosmétique, dermopharmaceutique ou pharmaceutique.

Les exemples qui suivent ont pour but d'illustrer la présente invention sans toutefois la limiter. Ils montrent que le nouveau latex inverse n'irrite pas la peau et que ses propriétés physiques permettent son utilisation dans la préparation de compositions cosmétiques, dermopharmaceutiques ou pharmaceutiques.

### Exemple 1 : Préparation et propriétés du latex inverse

### A Procédé de préparation

Comme décrit dans les demandes de brevet européen EP 0 186 361 et EP 0503 853, une émulsion eau dans huile, préparée à partir d'une solution aqueuse ajustée à pH = 5,8, comprenant les monomères 2-acrylamido-2-methyl propanesulfonique et acrylamide, et le méthylène bis(acrylamide), comme agent de réticulation et d'une solution organique comprenant de l'oléate de sorbitan commercialisé, par la demanderesse sous le nom MONTANE™ 80, et du WITCAMIDE™ 511 5 (un N,N-dialkanolamide d'acide gras partiellement estérifié), dissout dans de l'isohexadécane, est polymérisée puis puis additionnée de monooléate de sorbitan éthoxylé (20 OE), commercialisé par la demanderesse sous le nom MONTANOX™ 80. Le latex inverse obtenu est caractérisé par les mesures de viscosité suivantes (Température 20°C Brookfield LVT) :

| Dilution dans l'eau en % pondéral | Viscosité en mPas |
|---|---|
| 0,5 | ≈ 1 000 |
| 1,0 | ≈ 9 000 |
| 1,5 | ≈ 30 000 |
| 2,0 | ≈ 60 000 |
| 2,5 | ≈ 80 000 |
| 3,0 | ≈ 90 000 |

### B Propriétés

### a) Pouvoir stabilisant vis à vis des phases grasses

Le latex inverse préparé au paragraphe précédent (composition 1) a été utilisé pour préparer des émulsions avec différents types de corps gras, apolaires ou polaires, d'origine végétale ou synthétique. Les gels crèmes obtenus dans les différents cas sont stables et d'aspect parfaitement homogène. Leur viscosité est consignée dans le tableau suivant :

| Viscosité à 20°C, en mPa.s Brookfield LVT 6rpm | Huile utilisée pour la phase grasse du gel-crème (3% en composition 1 ; phase grasse: 20%) |
|---|---|
| ≈ 95 000 | Huile de jojoba |
| ≈ 95 000 | Huile d'amande douce |
| ≈ 90 000 | Squalane végétal |
| ≈ 80 000 | Diméthicone |
| ≈ 82 000 | Cyclométhicone |
| ≈ 92 000 | Isononyl isononanoate |
| ≈ 95 000 | Cetearyl octanoate |
| ≈. 92 000 | Benzoate C12-C15 |
| ≈ 90 000 | Caprylic / capric TG |

### b) stabilité à la température

On a préparé un gel crème comprenant 3% de composition 1 et 20% de cétéaryl octanoate et mesuré la viscosité Les résultats sont les suivants:

| | Viscosité Brookfield LVT 6rpm (en mPa.s) | |
|---|---|---|
| | A température ambiante | A 50°C |
| Après 1 jour | ≈ 90 000 | |
| Après 7 jours | | ≈ 90 000 |
| Après 1 mois | ≈ 87 000 | ≈ 90 000 |

### c) Influence du rayonnement UV sur la stabilité

On constate que le gel préparé avec la composition 1 est très stable UV; car sa viscosité n'a pas varié après 14 jours d'exposition.

### d) Influence du pH sur la viscosité

La viscosité du gel crème préparé avec la composition 1 est très stable au pH dans l'intervalle pH = 3 à pH = 8

### e) Etude comparative de la tolérance

On a comparé le latex inverse préparé selon le paragraphe A (composition 1), à une composition (composition 2), telle que décrite dans la demande de brevet européen EP 0503 853, comprenant une phase huile, une phase aqueuse, au moins un agent émulsifiant de type eau dans huile (E/H), au moins un agent émulsifiant de type huile dans eau (H/E), sous forme d'un latex inverse comprenant de 20% à 60% en poids, et de préférence de 25% à 45% en poids, d'un polyélectrolyte anionique, branché ou réticulé, à base d'acide 2-méthyl 2-[(1-oxo 2-propènyl) amino] 1-propanesulfonique partiellement ou totalement salifié, copolymérisé avec l'acrylamide, caractérisé en le solvant organique constitutif de la phase huile est de l'ISOPAR™ M, commercialisé par la société Exxon, qui est un mélange d'isoalcanes en C₁₁-C₁₅.

La tolérance cutanée a été déterminée selon la technique dite du test épicutané sous occlusion (patch test) sur l'homme. On a utilisé des cupules d'aluminium de 8 mm de diamètre et de 20 micro litres de capacité, chaque cupule permettant de couvrir une surface de 50 mm². Elles sont montées par paire sur un ruban adhésif. La solution aqueuse contenant 3% ou 5% des compositions 1 ou 2, imprègne des disques de papier-buvard, spécialement adaptés au système cupule/adhésif. Le système est appliqué sur la peau (région sous-capulaire gauche) des sujets. Quatorze volontaires, dont la moyenne d'âge est de 29 ans, ont servi de sujets. A titre de référence, on a également appliqué de la même façon sur chaque sujet mais sur une zone de peau différente, une solution à 2 % de laurylsulfate de sodium, ainsi que de l'eau distillée servant de témoin. 24 heures après l'application, les cupules sont retirées des zones sur lesquelles elles ont été appliquées. La lecture des résultats est réalisée 30 minutes, puis 24 heures après la dépose. Afin d'examiner si les produits appliqués sont tolérés ou non, on a considéré l'apparition des phénomènes suivants : érythème, oedème, vésicule, sécheresse cutanée, rugosité cutanée, réflectivité de la peau. Le pourcentage de tolérance parfaite cutanée, correspond au nombre de sujets n'ayant présenté aucun phénomène mentionné ci-dessus, par rapport au nombre total des sujets, à la lecture de 24 heures :

| | Composition 1 | | Composition 2 | |
|---|---|---|---|---|
| | 3% | 5% | 3% | 5% |
| Résultats à 24 heures (tolérance parfaite) | 100% | 95% | 55% | 18% |

Ces résultats montrent de façon inattendue que l'isohexadécane potentialise la tolérance cutanée du copolymère. Les exemples suivants mettent en oeuvre la composition 1 préparée au paragraphe A.

### Exemple 2 : Crème de soin

| | |
|---|---|
| Cyclométhicone | 10% |
| Composition 1 | 0,8% |
| MONTANOV™68 | 2% |
| alcool stéarylique | 1% |
| alcool stéarique | 0,5% |
| conservateur | 0,65% |
| Lysine | 0,025% |
| EDTA (sel disodique) | 0,05% |
| Gomme de xanthane | 0,2% |
| Glycérine | 3 % |
| Eau | q.s.p. 100% |

### Exemple 3 : Crème de soin

| | |
|---|---|
| Cyclométhicone | 10% |
| Composition 1 | 0,8% |
| MONTANOV ™ 68 | 2% |
| Perfluoropolymethylisopropylether | 0,5% |
| alcool stéarylique | 1% |
| alcool stéarique | 0,5% |
| conservateur | 0.65% |
| Lysine | 0,025% |
| EDTA (sel disodique) | 0,05% |
| PEMULEN™ TR | 0,2% |
| Glycérine | 3% |
| Eau | q.s.p. 100% |

### Exemple 4 : Baume après-rasage

### FORMULE

| | | |
|---|---|---|
| A | Composition 1 | 1,5% |
| | Eau | q.s.p 100% |
| B | MICROPEARL™ M 100 | 5,0% |
| | SEPICIDE™ CI | 0,50% |
| | Parfum | 0,20% |
| | éthanol 95° | 10,0% |

### MODE OPERATOIRE

Ajouter B dans A.

### Exemple 5 : Emulsion satinée pour le corps

### FORMULE

| | | |
|---|---|---|
| A | SIMULSOL™ 165 | 5,0% |
| | LANOL™ 1688 | 8,50% |
| | beurre de Karité | 2% |
| | huile de paraffine | 6,5% |
| | LANOL™ 14M | 3% |
| | LANOL™ S | 0,6% |
| | | |
| B | eau | 66.2% |
| | | |
| C | MICROPEARL™ M 100 | 5% |
| | | |
| D | Composition 1 | 3% |
| | | |
| E | SEPICIDE™ CI | 0,3% |
| | SEPICIDE™ HB | 0,5% |
| | MONTEINE™ CA | 1% |
| | Parfum | 0,20% |
| | acétate de vitamine E | 0,20% |
| | Sodium pyrolidinonecarboxylate | 1% (agent hydratant) |

### MODE OPERATOIRE

Ajouter C dans B, émulsionner B dans A à 70°C, puis ajouter D à 60°C puis E à 30°C.

### Exemple 6 : Lait corporel

### FORMULE

| | | |
|---|---|---|
| A | SIMULSOL™ 165 | 5,0% |
| | LANOL™ 1688 | 12,0% |
| | LANOL™ 14M | 2,0% |
| | alcool cétylique | 0,3% |
| | SCHERCEMOL™ OP | 3% |
| | | |
| B | eau | q.s.p. 100% |
| | | |
| C | Composition 1 | 0,35% |
| | | |
| D | SEPICIDE™ CI | 0,2% |
| | SEPICIDE™ HB | 0,5% |
| | Parfum | 0,20% |

### MODE OPERATOIRE

Emulsionner B dans A vers 75°C ; ajouter C vers 60°C, puis D vers 30°C

### Exemple 7 : Crème H/E

### FORMULE

| | | |
|---|---|---|
| A | SIMULSOL™ 165 | 5,0% |
| | LANOL™ 1688 | 20,0% |
| | LANOL™ P | 1,0% (additif à effet stabilisant) |
| | | |
| B | eau | q.s.p. 100% |
| | | |
| C | Composition 1 | 2,50% |
| | | |
| D | SEPICIDE™ CI | 0,20% |
| | SEPICIDE™ HB | 0,30% |

### MODE OPERATOIRE

Introduire B dans A vers 75°C ; ajouter C vers 60°C, puis D vers 45°C

### Exemple 8 : gel solaire non gras

### FORMULE

| | | |
|---|---|---|
| A | Composition 1 | 3,00% |
| | Eau | 30% |
| | | |
| B | SEPICIDE™ CI | 0,20% |
| | SEPICIDE™ HB | 0,30% |
| | Parfum | 0,10% |
| | | |
| C | colorant | q.s.p |
| | eau | 30% |
| | | |
| D | MICROPEARL™ M 100 | 3,00% |
| | Eau | q.s.p 100% |
| | | |
| E | huile de silicone | 2,0% |
| | PARSOL™ MCX | 5,00% |

### MODE OPERATOIRE

Introduire B dans A; ajouter C, puis D, puis E.

### Exemple 9 : Lait solaire

### FORMULE

| | | |
|---|---|---|
| A | SEPIPERL™ N | 3,0% |
| | huile de sésame | 5,0% |
| | PARSOL™ MCX | 5,0% |
| | Carraghénane λ | 0,10% |
| | | |
| B | eau | q.s.p.100% |
| | | |
| C | Composition 1 | 0,80% |
| | | |
| D | Parfum | q.s. |
| | Conservateur | q.s. |

### MODE OPERATOIRE

Emulsionner B dans A à 75°C puis ajouter C vers 60°C, puis D vers 30°C et ajuster le pH si nécessaire

### Exemple 10 : Gel de massage

### FORMULE

| | | |
|---|---|---|
| A | Composition 1 | 3,5% |
| | Eau | 20,0% |
| | | |
| B | colorant | 2 gouttes/100g |
| | Eau | q. s. |
| | | |
| C | alcool | 10% |
| | Menthol | 0,10% |
| | | |
| D | huile de silicone | 5,0% |

### MODE OPERATOIRE

Ajouter B dans A; puis ajouter au mélange, C puis D

### Exemple 11: gel soin de massage

### FORMULE

| | | |
|---|---|---|
| A | Composition 1 | 3,00% |
| | Eau | 30% |
| | | |
| B | SEPICIDE™ CI | 0,20% |
| | SEPICIDE™ HB | 0,30% |
| | Parfum | 0,05% |
| | | |
| C | colorant | q. s. |
| Eau | | q. s. p. 100% |
| | | |
| D | MICROPEARL™ SQL | 5,00% |
| | LANOL™ 1688 | 2% |

### MODE OPERATOIRE

Préparer A; additionner B, puis C, puis D.

### Exemple 12: Gel coup d'éclat

### FORMULE

| | | |
|---|---|---|
| A | Composition 1 | 4% |
| | Eau | 30% |
| B | ELASTINE HPM | 5,0% |
| C | NECROPEARL™ M 100 | 3% |
| | Eau | 5% |
| D | SEPICIDE™ CI | 0,2% |
| | SEPICIDE™ HB | 0,3% |
| | Parfum | 0,06% |
| Sodium pyrolidinonecarboxylate 50% | | 1% |
| Eau | | q. s. p. 100% |

### MODE OPERATOIRE

Préparer A; additionner B, puis C, puis D.

### Exemple 13: Lait corporel

### FORMULE

| | | |
|---|---|---|
| A | SEPIPERL™ N | 3,0% |
| | Triheptonate de glycérol | 10,0% |
| | | |
| B | eau | q.s.p.100% |
| | | |
| C | Composition 1 | 1,0% |
| | | |
| D | parfum | q. s. |
| | Conservateur | q. s. |

### MODE OPERATOIRE

Fondre A à environ 75°C . Emulsionner B dans A à 75°C puis ajouter C vers 60°C, puis D.

### Exemple 14 : Emulsion démaquillante à l'huile d'amande douce

### FORMULE

| | |
|---|---|
| MONTANOV™ 68 | 5% |
| huile d'amandes douces | 5% |
| eau | q.s.p.100% |
| Composition 1 | 0,3% |
| glycérine | 5% |
| conservateur | 0,2% |
| parfum | 03% |

### Exemple 15 : Crème hydratante pour peaux grasses

### FORMULE

| | |
|---|---|
| MONTANOV™ 68 | 5% |
| Cétylstéaryloctanoate | 8% |
| octyl palmitate | 2% |
| eau | q.s.p. 100% |
| Composition 1 | 0,6% |
| MICROPEARL™ M100 | 3,0% |
| Mucopolysaccharides | 5% |
| SEPICIDE™ HB | 0,8 |
| Parfum | 03% |

### Exemple 16: Baume après-rasage apaisant sans alcool

### FORMULE

| | |
|---|---|
| mélange de lauryl aminoacides | 0,1% à 5% |
| aspartate de magnésium et de potassium | 0,002% à 0,5% |
| LANOL™ 99 | 2% |
| huile d'amandes douces | 0,5% |
| eau | q.s.p.100% |
| Composition 1 | 3% |
| SEPICIDE™ HB | 0,3% |
| SEPICIDE™ CI | 0,2% |
| Parfum | 0,4% |

### Exemple 17 : Crème aux AHA pour peaux sensibles

### FORMULE

| | |
|---|---|
| mélange de lauryl aminoacides | 0,1% à 5% |
| aspartate de magnésium et de potassium | 0,002% à 0,5% |
| LANOL™ 99 | 2% |
| MONTANOV™ 68 | 5,0% |
| Eau | q.s.p.100% |
| Composition 1 | 1,50% |
| acide gluconique | 1,50% |
| tri éthylamine | 0,9% |
| SEPICIDE™ HB | 0,3% |
| SEPICIDE™ CI | 0,2% |
| Parfum | 0,4% |

### Exemple 18: Soin apaisant après soleil

### FORMULE

| | |
|---|---|
| mélange de lauryl aminoacides | 0,1% à 5% |
| aspartate de magnésium et de potassium | 0,002% à 0,5% |
| LANOL™ 99 | 10,0% |
| Eau | q.s.p.100% |
| Composition 1 | 2,50% |
| SEPICIDE™ HB | 0,3% |
| SEPICIDE™ CI | 0,2% |
| Parfum | 0,4% |
| Colorant | 0,03% |

### Exemple 19 : Lait démaquillant

### FORMULE

| | |
|---|---|
| SEPIPERL™ N | 3% |
| PRIMOL™ 352 | 8,0% |
| huile d'amandes douces | 2 % |
| eau | q.s.p.100% |
| Composition 1 | 0,8% |
| conservateur | 0,2% |

### Exemple 20 : Lait corporel

### FORMULE

| | |
|---|---|
| SEPIPERL™ N | 3,5% |
| LANOL™ 37T | 8,0% |
| SOLAGUM™ L | 0,05% |
| Eau | q.s.p.100% |
| Benzophénone | 2,0% |
| diméthicone 350cPs | 0,05% |
| Composition 1 | 0,8% |
| conservateur | 0,2% |
| parfum | 0,4% |

### Exemple 21 : Emulsion fluide à pH alcalin

| | |
|---|---|
| MARCOL™ 82 | 5,0% |
| NaOH | 10,0% |
| Eau | q.s.p.100% |
| Composition 1 | 1,5% |

### Exemple 22 : Fond de teint fluide

### FORMULE

| | |
|---|---|
| SIMULSOL™ 165 | 5,0% |
| LANOL™ 84D | 8,0% |
| LANOL™ 99 | 5,0% |
| Eau | q.s.p.100% |
| pigments et charges minérales | 10,0% |
| Composition 1 | 1,2% |
| conservateur | 0,2% |
| parfum | 0,4% |

### Exemple 23 : Lait solaire

### FORMULE

| | |
|---|---|
| SEPIPERL™ N | 3,5% |
| LANOL™ 37T | 10.0% |
| PARSOL NOX™ | 5,0% |
| EUSOLEX™ 4360 | 2,0% |
| Eau | q.s.p.100% |
| Composition 1 | 1,8% |
| conservateur | 0,2% |
| parfum | 0,4% |

### Exemple 24 : Gel contour des yeux

### FORMULE

| | |
|---|---|
| Composition 1 | 2,0% |
| Parfum | 0,06% |
| Sodium pyrroudinonecarboxylate | 0,2% |
| DOW CORNING™ 245 Fluid | 2,0% |
| Eau | q. s. p. 100% |

### Exemple 25: Composition de soin non rincée

### FORMULE

| | |
|---|---|
| Composition 1 | 1,5% |
| Parfum | q. s |
| Conservateur | q. s. |
| DOW CORNING™ X2 8360 | 5,0% |
| DOW CORNING™ Q2 1401 | 15,% |
| Eau | q.s.p. 100% |

### Exemple 26 : Gel amincissant

| | |
|---|---|
| Composition 1 | 5 % |
| Ethanol | 30 % |
| Menthol | 0,1 % |
| Caféine | 2,5 % |
| extrait de ruscus | 2 % |
| extrait de lierre | 2 % |
| SEPICIDE™ HP | 1 % |
| Eau | q. s. p. 100 % |

### Exemple 27 : Baume après-rasage apaisant sans alcool

### FORMULE

| | | |
|---|---|---|
| A | LIPACIDE™ PVB | 1,0% |
| | LANOL™ 99 | 2,0% |
| | Huile d'amandes douces | 0,5% |
| | | |
| B | Composition 1 | 3,5% |
| | | |
| C | eau | q.s.p.100% |
| | | |
| D | parfum | 0,4% |
| | SEPICIDE™ HB | 0,4% |
| | SEPICIDE™ CI | 0,2% |

### Exemple 28: Gel rafraîchissant après-rasage

### FORMULE

| | | |
|---|---|---|
| A | LIPACIDE™ PVB | 0,5% |
| | LANOL™ 99 | 5,0% |
| | Composition 1 | 2,5% |
| | | |
| B | eau | q.s.p.100% |
| | | |
| C | MICROPEARL™ LM | 0,5% |
| | Parfum | 0,2% |
| | SEPICIDE™ HB | 0,3% |
| | SEPICIDE™ CI | 0,2% |

### Exemple 29: Soin pour les peaux grasses

### FORMULE

| | | |
|---|---|---|
| A | MICROPEARL™ M310 | 1,0% |
| | Composition 1 | 5,0% |
| | Isononanoate d'octyle | 4.0% |
| | | |
| B | eau | q.s.p.100% |
| | | |
| C | SEPICONTROL™ A5 | 4,0% |
| | Parfum | 0,1% |
| | SEPICIDE™ HB | 0,3% |
| | SEPICIDE™ CI | 0,2% |
| | | |
| D | CAPIGEL™ 98 | 0,5% |
| | Eau | 10% |

### Exemple 30 : Crème aux AHA

### FORMULE

| | | |
|---|---|---|
| A | MONTANOV™ 68 | 5,0% |
| | LIPACIDE™ PVB | 1,05% |
| | LANOL™ 99 | 10,0% |
| | | |
| B | eau | q.s.p.100% |
| | Acide gluconique | 1,5% |
| | TEA (triéthylamine) | 0,9% |
| | | |
| C | Composition 1 | 1,5% |
| | | |
| D | parfum | 0,4% |
| | SEPICIDE™ HB | 0,2% |
| | SEPICIDE™ CI | 0,4% |

### Exemple 31 : Autobronzant non gras pour visage et corps

### FORMULE

| | | |
|---|---|---|
| A | LANOL™ 2681 | 3,0% |
| | Composition 1 | 2,5% |
| | | |
| B | eau | q.s.p.100% |
| | Dihydroxyacétone | 3,0% |
| | | |
| C | parfum | 0,2% |
| | SEPICIDE™ HB | 0,8% |
| | NaOH (hydroxyde de sodium) | qs pH = 5 % |

### Exemple 32 : Lait solaire au monoï de Tahiti

### FORMULE

| | | |
|---|---|---|
| A | Monoï de Tahiti | 10% |
| | LIPACIDE™ PVB | 0,5% |
| | Composition 1 | 2,2% |
| | | |
| B | eau | q.s.p.100% |
| | | |
| C | parfum | 0,1 % |
| | SEPICIDE™ HB | 0,3% |
| | SEPICIDE™ CI | 0,1% |
| | Méthoxycinnamate d'octyle | 4,0% |

### Exemple 33 : Soin solaire pour le visage

### FORMULE

| | | |
|---|---|---|
| A | Cyclométhicone et diméthiconol | 4,0% |
| | Composition 1 | 3,5% |
| | | |
| B | eau | q.s.p.100% |
| | | |
| C | parfum | 0,1% |
| | SEPICIDE™ HB | 0,3% |
| | SEPICIDE™ CI | 0,21% |
| | Méthoxycinnamate d'octyle | 5,0% |
| | Micatitane | 2,0% |
| | Acide lactique | q.s.p. pH = 6,5 |

### Exemple 34 : Emulsion bronzante sans soleil

### FORMULE

| | | |
|---|---|---|
| A | LANOL™ 99 | 15% |
| | MONTANOV™ 68 | 5,0% |
| | Paraméthoaycinnamate d'octyle | 3,0% |
| | | |
| B | eau | q.s.p.100% |
| | Dihydroxyacétone | 5,0% |
| | Phosphate monosodique | 0,2% |
| | | |
| C | Composition 1 | 0,5% |
| | | |
| D | parfum | 0,3% |
| | SEPICIDE™ HB | 0,8% |
| | NaOH | q.s. pH=5. |

Le MONTANOV™ 68 (cétéaryl glucoside), est une composition auto-émulsionnable telle que décrite dans WO 92/06778, commercialisée par la société SEPPIC.

Le MICROPEARL™ M 100 est une poudre ultra fine au toucher très doux et à action matifiante commercialisée par la société MATSUMO

Le SEPICIDE™ CI, imidazoline urée, est un agent conservateur commercialisé par la société SEPPIC.

PEMULEN™ TR est un polymère acrylique commercialisé par GOODRICH.

Le SIMULSOL™ 165 est du stéarate de glycérol auto-émulsionnable commercialisée par la société SEPPIC.

Le LANOL™ 1688 est un ester émollient à effet non gras commercialisé par la société SEPPIC.

Le LANOL™ 14M et le LANOL® S sont des facteurs de consistance commercialisés par la société SEPPIC.

Le SEPICIDE™ HB , qui est un mélange de phénoxyéthanol, de méthyl paraben, d'éthylparaben, de propylparaben et de butylparaben, est un agent conservateur commercialisé par la société SEPPIC.

Le MONTEINE™ CA est un agent hydratant commercialisé par la société SEPPIC.

Le SCHERCEMOL™ OP est un ester émollient à effet non gras.

Le LANOL™ P est un additif à effet stabilisant commercialisé par la société SEPPIC.

Le PARSOL™ MCX est de l'octyl paraméthoxycinnamate; commercialisé par la société GIVAUDAN.

Le SEPIPERL™ N est un agent nacrant, commercialisé par la société SEPPIC, à base d'un mélange d'alkyl poly glucosides tels que ceux décrits dans WO 95/13863.

Le MICROPEARL™ SQL est un mélange de micro particules renfermant du squalane qui se libère sous l'action du massage; il est commercialisé par la société MATSUMO.

Le LANOL™ 99 est de l'isononyl isononanoate commercialisé par la société SEPPIC.

Le LANOL™ 37T est du triheptanoate de glycérol, commercialisé par la société SEPPIC.

Le SOLAGUM™ L est un carraghénane commercialisé par la société SEPPIC.

Le MARCOL™ 82 est une huile de paraffine commercialisée par la société ESSO.

Le LANOL™ 84D est du malate de dioctyle commercialisé par la société SEPPIC.

Le PARSOL NOX™ est un filtre solaire commercialisé par la société GIVAUDAN.

l' EUSOLEX™ 4360 est un filtre solaire commercialisé par la société MERCK.

Le DOW CORNING™ 245 Fluid est de la cyclométhicone, commercialisée par la société DOW CORNING.

Le LIPACIDE™ PVB,est un hydrolysat de protéines de blé palmitoylé, est commercialisée par la société SEPPIC.

Le MICROPEARL™ LM est un mélange de squalane, de poly(méthylméthacrylate) et de Menthol, commercialisé par la société SEPPIC.

Le SEPICONTROL™ A5 est un mélange capryloy glycine, sarcosine, extrait de cinnamon zylanicum, commercialisé par la société SEPPIC. tel que ceux décrits dans la demande internationale de brevet PCT/FR98/01313 déposée le 23 juin 1998.

Le CAPIGEL™ 98 est un copolymère d'acrylates commercialisé par la société SEPPIC.

Le LANOL™ 2681 est un mélange caprylate, caprate de coprah, commercialisé par la société SEPPIC.

## Revendications

1. Composition cosmétique, dermopharmaceutique ou pharmaceutique, **caractérisée en ce qu'**elle comprend au moins une composition comprenant une phase huile, une phase aqueuse, au moins un agent émulsifiant de type eau dans huile (E/H), au moins un agent émulsifiant de type huile dans eau (H/E), sous forme d'un latex inverse comprenant de 20% à 60% en poids, et de préférence de 30% à 50% en poids, d'un polyélectrolyte anionique, branché ou réticulé, à base d'acide 2-méthyl 2-[(1-oxo 2-propènyl) amino] 1-propanesulfonique partiellement ou totalement salifié, copolymérisé avec l'acrylamide, le solvant organique constitutif de la phase huile dudit latex inverse étant de l'isohexadécane.

2. Composition cosmétique, dermopharmaceutique ou pharmaceutique, telle que définie à la revendication 1, **caractérisée en ce que** le polyélectrolyte anionique compris dans ledit latex inverse, comporte, en proportions molaires, de 30% à 50% de sel de sodium ou de sel d'ammonium de l'acide 2-méthyl 2-[(1-oxo 2-propènyl) amino] 1-propanesulfonique et de 50% à 70% d'acrylamide.

3. Composition cosmétique, dermopharmaceutique ou pharmaceutique, telle que définie à l'une quelconque des revendications 1 ou 2, **caractérisée en ce que** le poly électrolyte anionique compris dans ledit latex inverse, est réticulé et/ou branché avec un composé diéthylénique ou polyéthylénique dans la proportion molaire, exprimée par rapport aux monomères mis en oeuvre, de 0,005% à 1%, et de préférence de 0,01% à 0,2%.

4. Composition cosmétique, dermopharmaceutique ou pharmaceutique, telle que définie à la revendication 3, dans laquelle l'agent de réticulation et/ou l'agent de ramification dudit polyélectrolyte anionique, est choisi parmi le méthacrylate d'éthylène glycol, le diallyloxyacétate de sodium, le diacrylate d'éthylène glycol, le diallyl urée. le triméthylol propanetriacrylate ou le méthylène bis(acrylamide).

5. Composition cosmétique, dermopharmaceutique ou pharmaceutique, telle que définie à l'une quelconque des revendications 1 à 4, **caractérisée en ce que** ledit latex inverse, contient de 4% à 10% en poids, d'agents émulsifiants.

6. Composition cosmétique, dermopharmaceutique ou pharmaceutique, telle que définie à l'une quelconque des revendications 1 à 5, **caractérisée en ce que** la phase huile dudit latex inverse, représente de 15% à 40%, de préférence de 20% à 25%, du poids total dudit latex inverse.

7. Composition cosmétique, dermopharmaceutique ou pharmaceutique telle que définie à l'une quelconque des revendications 1 à 6, **caractérisée en ce que** ledit latex inverse contient en outre un ou plusieurs additifs choisis notamment parmi les agents complexant, les agents de transfert ou les agents limiteurs de chaînes.

8. Composition cosmétique, dermopharmaceutique ou pharmaceutique, telle que définie à l'une des revendications 1 à 7, comprenant de 0,1 % à 10 % en poids dudit latex inverse .

9. Composition cosmétique, dermopharmaceutique ou pharmaceutique telle que définie à la revendication 8, sous la forme d'un lait, d'une lotion, d'un gel, d'une crème, d'un savon, d'un bain moussant, d'un baume, d'un shampooing ou d'un après shampooing.

10. Utilisation d'une composition comprenant une phase huile, une phase aqueuse, au moins un agent émulsifiant de type eau dans huile (E/H), au moins un agent émulsifiant de type huile dans eau (H/E), sous forme d'un latex inverse comprenant de 20% à 60% en poids, et de préférence de 30% à 50% en poids, d'un polyélectrolyte anionique, branché ou réticulé, à base d'acide 2-méthyl 2-[(1-oxo 2-propènyl) amino] 1-propanesulfonique partiellement ou totalement salifié, copolymérisé avec l'acrylamide, le solvant organique constitutif de la phase huile dudit latex inverse étant de l'isohexadécane, pour préparer une composition cosmétique, dermopharmaceutique ou pharmaceutique.

11. Utilisation telle que définie à la revendication 10 pour préparer un lait, une lotion, un gel, une crème, un savon, un bain moussant, un baume, un shampooing ou un après shampooing.

## Patentansprüche

1. Kosmetische, dermopharmazeutische oder pharmazeutische Zusammensetzung, **dadurch gekennzeichnet, daß** sie mindestens eine Zusammensetzung, enthaltend eine Ölphase, eine wäßrige Phase, mindestens einen Wasser-in-Öl-Emulgator (W/O-Emulgator) und mindestens einen Öl-in-Wasser-Emulgator (O/W-Emulgator), in Form eines inversen Latex mit 20 bis 60 Gew.-% und vorzugsweise 30 bis 50 Gew.-% eines verzweigten oder vernetzten anionischen Polyelektrolyts auf Basis von teilweise oder vollständig in die Salzform überführter, mit Acrylamid copolymerisierter 2-Methyl-2-[(1-oxo-2-propenyl)amino]-1-propansulfonsäure enthält, wobei es sich bei dem die Ölphase des inversen Latex bildenden organischen Lösungsmittel um Isohexadecan handelt.

2. Kosmetische, dermopharmazeutische oder pharmazeutische Zusammensetzung nach Anspruch 1, **dadurch gekennzeichnet, daß** der in dem inversen Latex enthaltene anionische Polyelektrolyt 30 bis 50 Mol-% des Natriumsalzes oder des Ammoniumsalzes von 2-Methyl-2-[(2-oxo-2-propenyl)amino]-1-propansulfonsäure und 50 bis 70 Mol-% Acrylamid enthält.

3. Kosmetische, dermopharmazeutische oder pharmazeutische Zusammensetzung nach Anspruch 1 oder 2, **dadurch gekennzeichnet, daß** der in dem inversen Latex enthaltene anionische Polyelektrolyt mit einer diethylenischen oder polyethylenischen Verbindung in einem molaren Anteil, bezogen auf die eingesetzten Monomere, von 0,005 bis 1% und vorzugsweise 0,01 bis 0,2% vernetzt und/oder verzweigt ist.

4. Kosmetische, dermopharmazeutische oder pharmazeutische Zusammensetzung nach Anspruch 3, **dadurch gekennzeichnet, daß** das Vernetzungsmittel und/oder Verzweigungsmittel des anionischen Polyelektrolyten unter Ethylenglykolmethacrylat, Natriumdiallyloxyacetat, Ethylenglykoldiacrylat, Diallylharnstoff, Trimethylolpropantriacrylat und Methylen(bis)acrylamid ausgewählt ist bzw. sind.

5. Kosmetische, dermopharmazeutische oder pharmazeutische Zusammensetzung nach einem der Ansprüche 1 bis 4, **dadurch gekennzeichnet, daß** der inverse Latex 4 bis 10 Gew.-% Emulgatoren enthält.

6. Kosmetische, dermopharmazeutische oder pharmazeutische Zusammensetzung nach einem der Ansprüche 1 bis 5, **dadurch gekennzeichnet, daß** die Ölphase des inversen Latex 15 bis 40% und vorzugsweise 20 bis 25% des Gesamtgewichts des inversen Latex ausmacht.

7. Kosmetische, dermopharmazeutische oder pharmazeutische Zusammensetzung nach einem der Ansprüche 1 bis 6, **dadurch gekennzeichnet, daß** der inverse Latex außerdem auch noch ein oder mehrere Additive, die insbesondere unter Komplexbildnern, Kettenübertragungsmitteln und Kettenreglern ausgewählt sind, enthält.

8. Kosmetische, dermopharmazeutische oder pharmazeutische Zusammensetzung nach einem der Ansprüche 1 bis 7, die 0,1 bis 10 Gew.-% des inversen Latex enthält.

9. Kosmetische, dermopharmazeutische oder pharmazeutische Zusammensetzung nach Anspruch 8 in Form einer Milch, einer Lotion, eines Gels, einer Creme, einer Seife, eines Schaumbads, eines Balsams, eines Shampoos oder einer Haarpflegespülung.

10. Verwendung einer Zusammensetzung, enthaltend eine Ölphase, eine wäßrige Phase, mindestens einen Wasser-in-Öl-Emulgator (W/O-Emulgator) und mindestens einen Öl-in-Wasser-Emulgator (O/W-Emulgator), in Form eines inversen Latex mit 20 bis 60 Gew.-% und vorzugsweise 30 bis 50 Gew.-% eines verzweigten oder vernetzten anionischen Polyelektrolyts auf Basis von teilweise oder vollständig in die Salzform überführter, mit Acrylamid copolymerisierter 2-Methyl-2-[(1-oxo-2-propenyl)amino]-1-propansulfonsäure, wobei es sich bei dem die Ölphase des inversen Latex bildenden organischen Lösungsmittel um Isohexadecan handelt, zur Herstellung einer kosmetischen, dermopharmazeutischen oder pharmazeutischen Zusammensetzung.

11. Verwendung nach Anspruch 10 zur Herstellung einer Milch, einer Lotion, eines Gels, einer Creme, einer Seife, eines Schaumbads, eines Balsams, eines Shampoos oder einer Haarpflegespülung.

## Claims

1. Cosmetic, dermopharmaceutical or pharmaceutical composition, **characterized in that** it comprises an oil phase, an aqueous phase, at least one emulsifier of water-in-oil (W/O) type, at least one emulsifier of oil-in-water (O/W) type, in the form of an inverse latex comprising from 20% to 60% by weight and preferably from 30% to 50% by weight of a branched or crosslinked anionic polyelectrolyte based on partially or totally salified 2-methyl-2-[(1-oxo-2-propenyl)amino]-1-propanesulphonic acid, copolymerized with acrylamide, the organic solvent constituting the oil phase of said inverse latex being isohexadecane.

2. Cosmetic, dermopharmaceutical or pharmaceutical composition as defined in Claim 1, **characterized in that** the anionic polyelectrolyte included in said inverse latex comprises, in molar proportions, from 30% to 50% of the sodium salt or the ammonium salt of 2-methyl-2-[(1-oxo-2-propenyl)amino]-1-propanesulphonic acid and from 50% to 70% acrylamide.

3. Cosmetic, dermopharmaceutical or pharmaceutical composition as defined in either of Claims 1 and 2, **characterized in that** the anionic polyelectrolyte included in said inverse latex is crosslinked and/or branched with a diethylenic or polyethylenic compound in a molar proportion, expressed relative to the monomers used, of from 0.005% to 1% and preferably from 0.01% to 0.2%.

4. Cosmetic, dermopharmaceutical or pharmaceutical composition as defined in Claim 3, in which the crosslinking agent and/or branching agent of said anionic polyelectrolyte is chosen from ethylene glycol methacrylate, sodium diallyloxyacetate, ethylene glycol diacrylate, diallylurea, trimethylolpropane triacrylate and methylenebis(acrylamide).

5. Cosmetic, dermopharmaceutical or pharmaceutical composition as defined in any one of Claims 1 to 4, **characterized in that** said inverse latex contains from 4% to 10% by weight of emulsifiers.

6. Cosmetic, dermopharmaceutical or pharmaceutical composition as defined in any one of Claims 1 to 5, **characterized in that** the oil phase of said inverse latex represents from 15% to 40% and preferably from 20% to 25% of the total weight of said inverse latex.

7. Cosmetic, dermopharmaceutical or pharmaceutical composition as defined in any one of Claims 1 to 6, **characterized in that** said inverse latex also contains one or more additives chosen especially from complexing agents, transfer agents and chain-limiting agents.

8. Cosmetic, dermopharmaceutical or pharmaceutical composition as defined in one of Claims 1 to 7, comprising from 0.1% to 10% by weight of said inverse latex.

9. Cosmetic, dermopharmaceutical or pharmaceutical composition as defined in Claim 8, in the form of a milk, a lotion, a gel, a cream, a soap, a bubble bath, a balm, a shampoo or a conditioner.

10. Use of a composition comprising an oil phase, an aqueous phase, at least one emulsifier of water-in-oil (W/O) type, at least one emulsifier of oil-in-water (O/W) type, in the form of an inverse latex comprising from 20% to 60% by weight and preferably from 30% to 50% by weight of a branched or crosslinked anionic polyelectrolyte based on partially or totally salified 2-methyl-2-[(1-oxo-2-propenyl)amino]-1-propanesulphonic acid, copolymerized with acrylamide, the organic solvent constituting the oil phase of said inverse latex being isohexadecane, to prepare a cosmetic, dermopharmaceutical or pharmaceutical composition.

11. Use as defined in Claim 10, to prepare a milk, a lotion, a gel, a cream, a soap, a bubble bath, a balm, a shampoo or a conditioner.
